# EUROPEAN PATENT SPECIFICATION

(11) **EP 2 035 444 B1**
(45) Date of publication and mention of the grant of the patent: **02.09.2015**
(21) Application number: 07766553.7
(22) Date of filing: 15.06.2007
(51) Int. Cl.: C07K 5/06, A61K 38/05

(54) **DIPEPTIDE COMPOUNDS CONTAINING D-HISTIDINE**
D-HISTIDIN ENTHALTENDE DIPEPTIDVERBINDUNGEN
COMPOSÉS DIPEPTIDES CONTENANT D-HISTIDINE

(30) Priority: 23.06.2006 IT MI20061219
(43) Date of publication of application: 18.03.2009
(73) Proprietor: Flamma S.P.A., 24040 Chignolo d'Isola (IT)
(72) Inventor: NEGRISOLI, Gianpaolo, 24129 Bergamo (IT); CANEVOTTI, Renato, 24040 Chignolo D'Isola (IT); PREVITALI, Massimo, 24040 Chignolo D'Isola (IT)
(74) Representative: Minoja, Fabrizio
(86) International application number: PCT/IB2007/001605
(87) International publication number: WO 2008/001175

(56) References cited:
- EP-A- 0 500 332
- EP-A- 1 297 830
- WO-A1-2005/092850
- CACCIATORE I ET AL: "Biochemical properties of new synthetic carnosine analogues containing the residue of 2,3-diaminopropionic acid: the effect of N-acetylation" AMINO ACIDS, SPRINGER VERLAG, AU, vol. 28, 18 January 2005 (2005-01-18), pages 77-83, XP002452851 ISSN: 0939-4451
- BABIZHAYEV ET AL: "Biological activities of the natural imidazole-containing peptidomimetics n-acetylcarnosine, carcinine and l-carnosine in ophthalmic and skin care products" LIFE SCIENCES, PERGAMON PRESS, OXFORD, GB, vol. 78, no. 20, 11 April 2006 (2006-04-11), pages 2343-2357, XP005357350 ISSN: 0024-3205

## Description

The present invention relates to dipeptide derivatives containing D-histidine, and compositions containing them.

Cigarette smoke contains a mixture of carbonyl compounds including acetaldehyde (1110-2101 µg/cigarette), acrolein (238-468 µg/cigarette), formaldehyde (87.0-243 µg/cigarette), propanal (87.0-176 µg/cigarette), malondialdehyde (18.9-36.0 µg/cigarette), methylglyoxal (13.4-59.6 µg/cigarette) and glyoxal (1.93-6.98 µg/cigarette) (Fujioka K. et al., Environ Toxicol. 2006 Feb; 21(1):47-54).

Some of these aldehydes are highly reactive and cytotoxic, such as acrolein, an alpha, beta-unsaturated aldehyde characterised by an electrophilic C3, and the dialdehydes glyoxal and methylglyoxal (O' Brien PJ. et al., Crit Rev Toxicol. 2005 Aug; 35(7):609-62). In particular, acrolein reacts with the bases of DNA to form hexocyclic adducts which can play a leading role in the mutagenesis and carcinogenesis induced by cigarette smoke (Cohen SM. et al., Cancer Res. 1992 Jul 1;52(13):3577-81; Yang IY. et al., Biochemistry. 2002 Nov 19;41(46):13826-32; Yang IY. et al., Biochemistry. 2002; 41 (46):13826-32). Acrolein also has many other harmful biological effects, including: 1) a rapid increase in vascular nitric oxide which involves a process of apoptosis affecting the endothelial cells; said process may partly explain the angiopathy induced by cigarette smoke (Misonou Y. et al., Nitric Oxide. 2006 Mar; 14(2); 180-7); 2) inhibition of T-cell response with a consequent immunosuppression effect (Lambert C. et al., J Allergy Clin Immunol. 2005 Oct; 116(4):916-22); c) a functional alteration of proteins and peptides by formation of covalent adducts and cross-bridges (Uchida K. Trends Cardiovasc Med. 1999 Jul; 9(5):109-13).

The lung tissue of smokers is also subjected to an oxidative process induced by oxidising species contained in the smoke, such as nitrogen dioxide; these species are responsible for the lipoperoxidative process affecting polyunsaturated fatty acids, which entails the formation of a second group of aldehydes, also highly cytotoxic and reactive, such as 4-hydroxy-trans-nonenal (HNE) (2: Iles KE. et al., Free Radic Biol Med. 2005 Aug 1;39(3):355-64). Like acrolein, HNE is an alpha, beta-unsaturated aldehyde able to form covalent adducts with DNA, inducing mutagenesis and carcinogenesis (Kowalczyk P. et al., Mutat Res. 2004 Jun 4;550(1-2):33-48) and involved in various molecular processes which lead to cell dysfunction and damage to the lung tissue. A significant correlation between the HNE content of the pulmonary epithelium and the lung function has also been observed (Rahman I. et al., Am J Respir Crit Care Med. 2002 Aug 15;166(4):490-5).

As the lung tissue of smokers is continually exposed to alpha, beta-unsaturated aldehyde compounds (present in smoke or induced by it) and these compounds are partly responsible for mutagenic, carcinogenic and tissue dysfunction processes, an increase in detoxification processes aimed at the α , β -unsaturated aldehydes in the lung tissue would potentially represent a preventive pharmacological approach.

Earlier studies have demonstrated that carnosine (β -alanyl-L-histidine) performs an important specific detoxifying role towards the α , β-unsaturated aldehydes, with the formation of the corresponding Michael adduct (due to the reaction of the C3 of HNE with the Nτ of the imidazole ring) which lacks reactivity, and consequently cytotoxicity (Aldini G. et al., Biofactors. 2005; 24(1-4):77-87; Aldini G. et al., Biochem Biophys Res Commun. 2002 Nov 15;298(5):699-706). This detoxification mechanism was recently demonstrated in muscle tissue which had suffered oxidative damage (Orioli M. et al., J Chromatogr B Analyt Technol Biomed Life Sci. 2005 Nov 15;827(1):109-18).

Carnosine which, together with glutathione, can be involved in detoxification of the alpha, beta-unsaturated aldehydes induced by/present in cigarette smoke, is not present in lung tissue but is present in high concentrations in other tissues, such as the skeletal muscle, myocardium and some parts of the brain (Jackson MC. et al., Inflamm Res. 1996 Mar; 45(3):132-5). The lack of carnosine in the lung tissue can be explained by the presence of carnosinases which are responsible for the hydrolytic process of the dipeptide, with the formation of the two constituting aminoacids (Lenney JF. Biochim Biophys Acta. 1976 Mar 11;429(1):214-9).

Surprisingly, we recently demonstrated in the rat that D-carnosine (β-alanyl-D-histidine), a peptidomimetic of L-carnosine which is resistant to carnosinase, is distributed in the lung after oral administration. It has also been demonstrated that D-carnosine performs the same detoxification of the alpha, beta-unsaturated carbonyl compounds of L-carnosine. However, the therapeutic application of D-carnosine is considerably limited by its poor gastrointestinal absorption, as recently demonstrated by pharmacokinetic studies conducted on laboratory animals by monitoring the plasma carnosine content with a liquid chromatography test interfaced via an ESI source with a triple quadruple mass spectrometer.

### Description of the invention

The present invention relates to lipophilic derivatives of D-carnosine, which are characterised by greater bioavailability than L-carnosine, and are distributed in the lung, where they detoxify the cytotoxic carbonyl compounds induced by cigarette smoke.

The compounds of the invention have general formula I 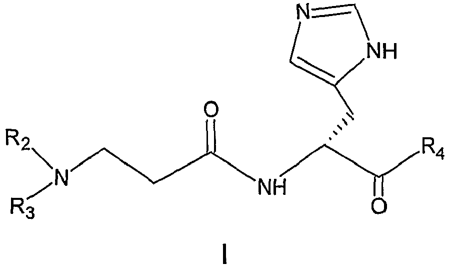 in which:
R₂ and R₃ which can be the same or different, are:
   hydrogen, except when R₄ represents a hydroxy group;
   a straight or branched C₁-C₂₀ or cyclic C₃-C₇ alkylcarbonyl group optionally containing one or more double bonds, except for the acetyl group when R₄ represents a hydroxy group;
   an arylcarbonyl or arylalkylcarbonyl group;
   a straight or branched C₁-C₁₀ or cyclic C₃-C₇ alkoxycarbonyl group optionally containing one or more double bonds;
   an arylalkoxycarbonyl group;
   an amino group;
   a hydroxy group;
   a group of general formula IV 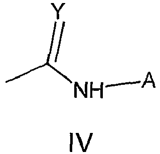 in which Y is nitrogen, oxygen or sulfur and A is hydrogen or an
      amino group;
R₄ is:
   a hydroxy group, except when R₂ or R₃ represent hydrogen or an acetyl group;
R₄ is:
   a hydroxy group, except when R₂ or R₃ represent hydrogen or an acetyl group;
   an optionally mono or disubstituted amino group;
   a straight or branched C₁-C₂₀ or cyclic C₃-C₇ alkyl group, optionally containing one or more double bonds;
   an arylalkyl group;
   a straight or branched C₁-C₂₀, preferably C₄-C₁₀, or cyclic C₃-C₇, alkoxy group optionally containing one or more double bonds;
   an alkoxycarbonyloxyalkoxy or alkylcarbonyloxyalkoxy group bearing a straight, branched or cyclic alkyl;
   an arylalkoxy group.

The invention also comprises the pharmaceutically acceptable salts of the compounds of general formula 1.

The aryl moieties of the aryl, aryl-alkyl, arylcarbonyl, arylalkylcarbonyl, aryloxycarbonyl, arylalkyloxycarbonyl, aryloxy, arylalkoxy groups defined above can be mono- or polycyclic and optionally substituted with one or more substituents selected from:
hydroxy, C₁-C₅-alkoxy, C₁-C₅ -alkoxycarbonyl groups;
amino, C₁-C₅ -mono- or di-alkylamino, C₁-C₅-acylamino groups;
halogen atoms such as Cl, Br, F, I;
straight, branched or cyclic alkyl groups;
optionally substituted aryl groups.

Preferred alkyl, alkoxy or alkylcarbonyl groups are those in which the alkyl residue is represented by methyl, ethyl, propyl, isopropyl, n-butyl, sec-butyl, tert-butyl, n-pentyl, n-hexyl, n-octyl, n-decyl, n-hexadecyl.

Preferred cycloalkyl groups are cyclopropyl, cyclopentyl and methoxy, amino, dimethylamino, methylamino, ethylamino, diethylamino, acetylamino, formylamino, propionylamino, butanoylamino groups, or halogen atoms, is meant.

A preferred aralkyl group is benzyl or phenethyl.

More particularly preferred compounds are D-carnosine C₄-C₁₀ esters.

The compounds of the invention are characterized in that the configuration of the chiral center bearing the methylimidazole substituent has to be (R), while any other chiral centers present can have either (R) or (S) configuration, depending on the compounds.

For the intended uses, the compounds of formula I are conveniently formulated in conventional pharmaceutical, cosmetic or nutritional compositions, suitable for the administration through the oral, parenteral, topical or transdermal route. Said compositions are a further object of the invention. Examples of said compositions are capsules, tablets, syrups, injectable solutions or suspensions, ointments, suppositories, controlled-release forms and the like, water-soluble granulates. Said forms, in addition to the carriers and excipients used in the pharmaceutical technique, may optionally contain other active ingredients having complementary or anyway useful activity for the treatment/prevention of the concerned pathologies.

The compounds of the invention can be prepared using well known techniques, starting from the suitable D-histidine and 3-aminopropionic intermediates in which residues R₂, R₃, R₄ have the meanings defined above in the case of the molecules of formula (I), or starting from 3-aminopropionyl-D-histidine for the subsequent introduction of the necessary substituents.

For example, the compounds of the invention can be prepared by coupling 3-aminopropionic derivatives, suitably substituted at the nitrogen, with D-histidine derivatives bearing the appropriate R₄ substituent, optionally suitably protected, using a coupling method as those described for example in Houben Weil "Synthesis of peptides and peptidomimetics" E 22a chapter 3.

Alternatively, the compounds (I) can be prepared starting from 3-aminopropionyl-D-histidine by subsequent introduction of the necessary substituents using established procedures, such as those reported in T.Greene, P. Wuts "Protective Groups in Organic Chemistry" for functionalization of the carboxylic and amino groups.

### Experimental section

### D-Carnosine Ethyl Ester Hydrochloride

0.8 g of D carnosine are placed in a 50 ml two-necked round-bottom flask; dissolved in EtOH (16 ml), then added with one drop of DMSO and 2 ml of HCl-saturated Et₂O.

The mixture is cooled to 5°C on an ice bath, then 0.46 g of thionyl chloride are slowly dropped therein (temperature should not exceed 10°C). The mixture is refluxed at 60°C, monitoring the progress of the reaction by TLC. After completion of the reaction, the mixture is left to cool at room temperature and the solvent is evaporated off under reduced pressure.

Final residue: 1.25 g, M.W. 363,6, yield 97%.
¹H-NMR (DMSO-D6): ppm 1,17 t 3H; 2,55 t 2H; 2,95 m 2H; 3,0-3,2 m 2H; 4,1 q 2H; 4,55 m 1H; 7,4 s 1H; 8,78 d 1H.

### D-Carnosine Octyl Ester Hydrochloride

0.7 g of D-carnosine are loaded in a round-bottom flask equipped with magnetic stirring; 15 ml of 1-octanol, and 1.75 ml of HCl-saturated Et₂O are added. The mixture is cooled to 5°C on an ice bath, then 0.48 g of thionyl chloride are added dropwise (temperature should not exceed 10°C).

The mixture is then heated to 90°C, monitoring the progress of the reaction by TLC. After completion of the reaction, the mixture is left to cool at room temperature and the solvent is distilled off (T = 30°C, P = 1 mmHg).

The resulting residue is taken up into hexane, to obtain 1.11 g of a white solid which is filtered.
¹H-NMR (DMSO-D6) ppm 0.8 t 3H; 1,2 m 10H; 1,45 m 2H; 2,3 t 2H; 2,85 m 2H; 1,9-3,2 dq 2H; 3,95 t 2H; 4,52 m 1 H; 7,40 s 1 H; 8,75 d 1 H.

### D-Carnosine Methyl Ester Hydrochloride

A 50 ml reactor is loaded with 0.6 g of D-carnosine dissolved in methanol (12 ml), then one drop of DMSO and 1.5 ml of HCl-saturated Et₂O are added. The mixture is cooled to 5°C on an ice bath, then 0.2 ml of thionyl chloride are added dropwise (temperature should not exceed 10°C). The mixture is then heated to 60°C, monitoring the progress of the reaction by TLC. After completion of the reaction, the solvent is evaporated off under reduced pressure to obtain 0.88 g of a clear oil.
¹H-NMR (DMSO-D6): ppm 2,55 t 2H; 2,95 m 2H; 3,05-3,2 m 2H; 3,65 s 3H; 4,1 m 1H; 7,4 s 1H; 8,8 d 1H.

### N-Acetyl-D-Carnosine Ethyl Ester

1 g of D-carnosine ethyl ester hydrochloride is placed in a round-bottom flask and dissolved in EtOH (60 ml). The solution is added with 0.42 g of acetic anhydride and 1.38 g of potassium bicarbonate and the mixture is stirred at room temperature, monitoring the progress of the reaction by TLC. After completion of the reaction, the solid is removed and the solvent is evaporated off. The final residue is treated with acetone, thereby obtaining a white residue which is filtered off, whereas the solvent is evaporated, to obtain 0.33 g of a semisolid residue.
¹H-NMR(DMSO-D6): ppm 1,1 t 3H; 1,75 s 3H; 2,28 t 2H; 2,81- 2,97 m 2H; 3,18 m 2H; 4,05 q 2H; 4,4 m 1H; 7,5 s 1H; 8,45 m 1H.

The following compounds are prepared analogously:

### N-Acetyl-D-Carnosine Methyl Ester

¹H-NMR (DMSO-D6): ppm 1,78 s 3H; 2,15 t 2H; 2,78-2,95 m 2H; 3,17 m 2H; 3,58 s 3H; 4,4 m 1H; 7,4 s 1H; 8,6 d 1H.

### N-Acetyl-D-Carnosine Octyl Ester

¹H-NMR (CD3OD): ppm 1,85 t 3H; 2,28 m 10H; 2,55 m 2H; 2,90 s 3H; 3,35 t 3H; 3,9-4,1 dq 2H; 4,35 m 2H; 5,05 m 2H; 5,65 m 1 H; 7,85 s 1 H; 8,60 s 1H.

### N-Eoc-D-Carnosine

A round-bottom flask is loaded with 2 g of D-carnosine and 30 ml of absolute ethanol, then 2.7 g of bicarbonate are added and the solution is cooled a 5°C. Subsequently, 1.1 g of ethyl chloroformate dissolved in 5 ml of THF are dropped therein. The mixture is warmed to room temperature and reacted for an hour at this temperature, then for 3 hours at 50°C. Inorganic salts are filtered off and solvent is evaporated off under reduced pressure to obtain 2.17 g of a spongy solid.
¹H-NMR (DMSO-D6) ppm 1,15 t 3H; 2,25 t 2H; 2,80-3,05 dq 2H; 3,15 q 2H; 3,95 q 2H; 4,42 m 1 H; 6,85 s 1 H; 7,75 s 1 H.

The following compound is prepared analogously:

### N-Eoc-β -Alanine

¹H-NMR (DMSO-D6) ppm 1,12 t 3H; 2,35 t 2H; 3,18 q 2H; 3,95 q 2H; 7,05 br. s 1H.

### N-Eoc-D-Carnosine Methyl Ester

A 50 ml two-necked round-bottom flask is loaded with 1 g of D-histidine methyl ester hydrochloride, which is dissolved in about 10 ml of dry DMF, then 0.73 g of N-methylmorpholine are added and temperature is brought to 0°C.

After that, 0.7 g of N-Eoc-β -alanine are added and 0.9 g of DCC dissolved in 3 ml of dry DMF are dropwise added. The mixture is reacted at 0°C for about three hours, left at 5°C overnight, then filtered and the solvent is evaporated off.

The residue is taken up in chloroform and washed with a sodium bicarbonate saturated solution (3x30 ml) and water. The organic phase is then dried and evaporated.

The resulting residue is purified by silica gel chromatography, using a CH₂Cl₂/CHCl₃ 95/5 mixture as the eluent.

The pure fractions are combined and the solvent is evaporated off to obtain 1 g of a solid spongy residue.
¹H-NMR (CDCl₃) ppm 1,22 t 3H; 2,42 t 2H; 3,1 m 2H; 3,45 m 2H; 3,70 s 3H; 4,10 q 2H; 4,80 m 1H; 6,80 s 1H; 7,6 s 1H.

The following compound is prepared analogously:

### N-Eoc-D-Carnosine Benzyl Ester

¹H-NMR (CDCl3) ppm: 1,32 t 3H; 2,42 t 2H; 3,1 d 2H; 3,45 m 2H; 4,10 Q 2H; 4,35 m 1H; 5,09-5,18 dd 2H; 6,52 s 1H; 7,12 d 1H;7,30-7,40 m 4H; 7,52 s 1H.

## Claims

1. Compounds of general formula (I) 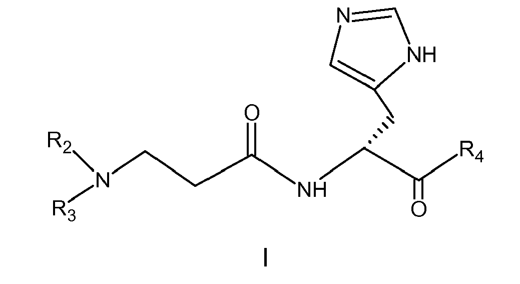 in which:
R₂ and R₃ which can be the same or different, are:
hydrogen, except when R₄ represents a hydroxy group;
a straight or branched C₁-C₂₀ or cyclic C₃-C₇ alkylcarbonyl group optionally containing one or more double bonds, except for the acetyl group when R₄ represents a hydroxy group;
an arylcarbonyl or arylalkylcarbonyl group;
a straight or branched C₁-C₁₀ or cyclic C₃-C₇ alkoxycarbonyl group optionally containing one or more double bonds;
an arylalkoxycarbonyl group;
an amino group;
a hydroxy group;
a group of general formula IV 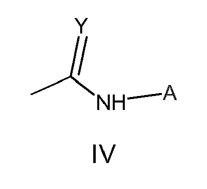 in which Y is nitrogen, oxygen or sulfur and A is hydrogen or an amino group;
R₄ is:
a hydroxy group, except when R₂ or R₃ represent hydrogen or an acetyl group;
an optionally mono or disubstituted amino group;
a straight or branched C₁-C₂₀ or cyclic C₃-C₇ alkyl group, optionally containing one or more double bonds;
an arylalkyl group;
a straight or branched C₁-C₂₀, preferably C₄-C₁₀, or cyclic C₃-C₇,
alkoxy group optionally containing one or more double bonds;
an alkoxycarbonyloxyalkoxy or alkylcarbonyloxyalkoxy group bearing a straight, branched or cyclic alkyl;
an arylalkoxy group;
the pharmaceutically acceptable salts thereof.

2. Compounds as claimed in claim 1 wherein
R₂ and R₃ which can be the same or different, are:
hydrogen, except when R₄ represents a hydroxy group;
a straight or branched C₁-C₂₀ or cyclic C₃-C₇ alkylcarbonyl group optionally containing one or more double bonds, with the exclusion of the acetyl group when R₄ represents a hydroxy group;
an arylcarbonyl or arylalkylcarbonyl group;
a straight or branched C₁-C₁₀ or cyclic C₃-C₇ alkoxy group optionally containing one or more double bonds;
an arylalkoxycarbonyl group;
an amino group;
R₄ is:
a hydroxy group, except when R₂ or R₃ represent hydrogen or an acetyl group;
a straight or branched C₁-C₂₀ or cyclic C₃-C₇ alkoxy group optionally containing one or more double bonds;
an alkoxycarbonyloxyalkoxy or alkylcarbonyloxyalkoxy group bearing a straight, branched or cyclic alkyl;
an arylalkoxy group.

3. Compounds as claimed in claim 2 in which
R₄ is:
a hydroxy group, except when R₂ or R₃ represent hydrogen or an acetyl group,
a straight or branched C₄-C₁₆ or cyclic C₃-C₇ alkoxy group, optionally containing one or more double bonds.

4. Compounds as claimed in claim 3 in which R₄ is a straight or branched C₄-C₁₀ alkoxy group.

5. A compound as claimed in claims 1-3 selected from:
D-carnosine ethyl ester and the salts thereof
D-carnosine octyl ester and the salts thereof
D-carnosine methyl ester and the salts thereof
D-carnosine n-butil ester and the salts thereof
N-acetyl-D-carnosine ethyl ester
N-acetyl-D-carnosine methyl ester
N-acetyl-D-carnosine octyl ester
N-Eoc-D-carnosine and the salts thereof
N-Eoc-D-carnosine methyl ester
N-Eoc-D-carnosine benzyl ester
D-carnosine -1-(etoxycarbonyloxy)ethyl ester
Eoc- D-carnosine-1- (etoxycarbonyloxy)ethyl ester
Z-D carnosine and the salts thereof
Z-D carnosine methyl ester
Z-D carnosine octyl ester

6. A composition containing an effective amount of one or more compounds as claimed in claims 1-5 in combination with suitable excipients.

## Patentansprüche

1. Verbindungen der allgemeinen Formel (I) 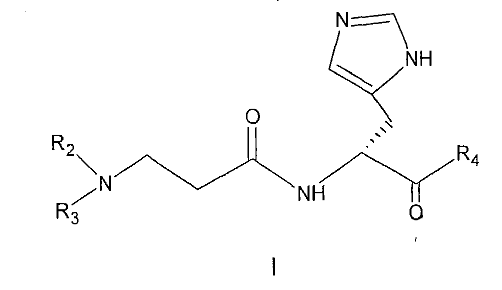 in der:
R₂ und R₃, die gleich oder verschieden sein können, sind:
Wasserstoff, ausgenommen wenn R₄ für eine Hydroxygruppe steht;
eine geradkettige oder verzweigte C₁-C₂₀- oder cyclische C₃-C₇-Alkylcarbonylgruppe, die gegebenenfalls eine oder mehrere Doppelbindungen enthalten, ausgenommen für die Acetylgruppe, wenn R₄ für eine Hydroxygruppe steht;
eine Arylcarbonyl- oder Arylalkylcarbonylgruppe;
eine geradkettige oder verzweigte C₁-C₁₀- oder cyclische C₃-C₇-Alkoxycarbonylgruppe, die gegebenenfalls eine oder mehrere Doppelbindungen enthalten;
eine Arylalkoxycarbonylgruppe;
eine Aminogruppe;
eine Hydroxygruppe;
eine Gruppe der allgemeinen Formel IV 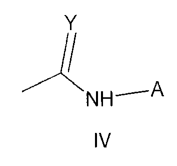 in der Y Stickstoff, Sauerstoff oder Schwefel ist und A Wasserstoff oder eine Aminogruppe ist;
R₄ ist:
eine Hydroxygruppe, ausgenommen wenn R₂ oder R₃ für Wasserstoff oder eine Acetylgruppe stehen;
eine gegebenenfalls mono- oder disubstituierte Aminogruppe;
eine geradkettige oder verzweigte C₁- C₂₀- oder cyclische C₃-C₇-Alkylgruppe, die gegebenenfalls eine oder mehrere Doppelbindungen enthalten; eine Arylalkylgruppe;
eine geradkettige oder verzweigte C₁-C₂₀-, vorzugsweise C₄-C₁₀, oder cyclische C₃-C₇- Alkoxygruppe, die gegebenenfalls eine oder mehrere Doppelbindungen enthalten;
eine Alkoxycarbonyloxyalkoxy- oder Alkylcarbonyloxyalkoxygruppe, welche ein geradkettiges, verzweigtes oder cyclisches Alkyl tragen;
eine Arylalkoxygruppe;
die pharmazeutisch akzeptablen Salze davon.

2. Verbindungen nach Anspruch 1, wobei
R₂ und R₃, die gleich oder verschieden sein können, sind:
Wasserstoff, aufgenommen wenn R₄ für eine Hydroxygruppe steht;,
eine geradkettige oder verzweigte C₁-C₂₀- oder cyclische C₃-C₇-Alkylcarbonylgruppe, die gegebenenfalls eine oder mehrere Doppelbindungen enthalten, mit Ausnahme der Acetylgruppe, wenn R₄ für eine Hydroxygruppe steht;
eine Arylcarbonyl- oder Arylalkylcarbonylgruppe;
eine geradkettige oder verzweigte C₁-C₁₀- oder cyclische C₃-C₇-Alkoxygruppe,
die gegebenenfalls eine oder mehrere Doppelbindungen enthalten;
eine Arylalkoxycarbonylgruppe;
eine Aminogruppe;
R₄ ist:
eine Hydroxygruppe, ausgenommen wenn R₂ oder R₃ für Wasserstoff oder eine Acetylgruppe stehen;
eine geradkettige oder verzweigte C₁-C₂₀- oder cyclische C₃-C₇-Alkoxygruppe die gegebenenfalls eine oder mehrere Doppelbindungen enthalten; ,
eine Alkoxycarbonyloxyalkoxy- oder Alkylcarbonyloxyalkoxygruppe, die eine geradkettige, verzweigte oder cyclische Alkylgruppe tragen;
eine Arylalkoxygruppe.

3. Verbindungen nach Anspruch 2, in denen
R₄ ist:
eine Hydroxygruppe, ausgenommen, wenn R₂ oder R₃ für Wasserstoff oder eine Acetylgruppe stehen,
eine geradkettige oder verzweigte C₄-C₁₆- oder cyclische C₃-C₇-Alkoxygruppe, die gegebenenfalls eine oder mehrere Doppelbindungen enthalten.

4. Verbindungen nach Anspruch 3, in denen R₄ eine geradkettige oder verzweigte C₄-C₁₀-Alkoxygruppe ist.

5. Verbindung nach Anspruch 1-3, ausgewählt aus:
D-Carnosinethylester und Salze davon
D-Carnosinoctylester und Salze davon
D-Carnosinmethylester und Salze davon
D-Carnosin-n-butylester und Salze davon
N-Acetyl-D-carnosinethylester
N-Acetyl-D-carnosinmethylester
N-Acetyl-D-carnosinoctylester
N-Eoc-D-carnosin und Salze davon
N-Eoc-D-carnosinmethylester
N-Eoc-D-carnosinbenzylester
D-Carnosin-1-(ethoxycarbonyloxy)ethylester
Eoc-D-Carnosin-1-(ethoxycarbonyloxy)ethylester Z-D-Carnosin und Salze davon
Z-D-Carnosinmethylester
Z-D-Carnosinoctylester

6. Verbindung, die eine wirksame Menge einer oder mehrerer Verbindungen nach Anspruch 1 - 5 in Kombination mit geeigneten Exzipientien enthält.

## Revendications

1. Composés de formule générale (I) 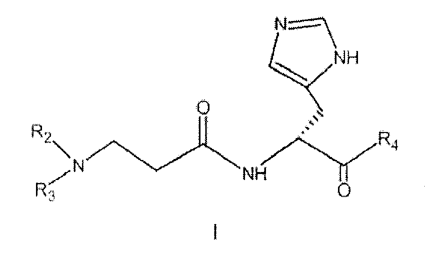 dans laquelle :
R₂ et R₃ qui peuvent être identiques ou différents, sont :
un atome d'hydrogène, excepté lorsque R₄ représente un groupe hydroxyle ;
un groupe alkylcarbonyle en C₁ à C₂₀ linéaire ou ramifié ou en C₃ à C₇ cyclique contenant éventuellement une ou plusieurs doubles liaisons, excepté pour le groupe acétyle lorsque R₄ représente un groupe hydroxyle ;
un groupe arylcarbonyle ou un groupe arylalkylcarbonyle ;
un groupe alcoxycarbonyle en C₁ à C₁₀ linéaire ou ramifié ou en C₃ à C₇ cyclique contenant éventuellement une ou plusieurs doubles liaisons ;
un groupe arylalcoxycarbonyle ;
un groupe amino ;
un groupe hydroxyle ;
un groupe de la formule générale IV 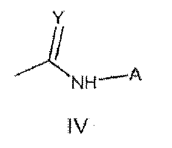 dans laquelle Y est un atome d'azote, un atome d'oxygène ou un atome de soufre et A est un atome d'hydrogène ou un groupe amino ;
R₄ est :
un groupe hydroxyle, excepté lorsque R₂ ou R₃ représente un atome d'hydrogène ou un groupe acétyle ;
un groupe amino éventuellement mono ou disubstitué ;
un groupe alkyle en C₁ à C₂₀ linéaire ou ramifié ou en C₃ à C₇ cyclique, contenant éventuellement une ou plusieurs doubles liaisons ;
un groupe arylalkyle ;
un groupe alcoxy en C₁ à C₂₀ linéaire ou ramifié, de préférence en C₄ à C₁₀, ou en C₃ à C₇ cyclique, contenant éventuellement une ou plusieurs doubles liaisons ;
un groupe alcoxycarbonyloxyalcoxy ou alkylcarbonyloxyalcoxy portant un groupe alkyle linéaire, ramifié ou cyclique ;
un groupe arylalcoxy :
leurs sels pharmaceutiquement acceptables.

2. Composés tels que revendiqués selon la revendication 1 dans lequel
R₂ et R₃ qui peuvent être identiques ou différents, sont :
un atome d'hydrogène, excepté lorsque R₄ représente un groupe hydroxyle ;
un groupe alkylcarbonyle en C₁ à C₂₀ linéaire ou ramifjé ou en C₃ à C₇ cyclique contenant éventuellement une ou plusieurs doubles liaisons, à l'exclusion du groupe acétyle lorsque R₄ représente un groupe hydroxyle ;
un groupe arylcarbonyle ou arylalkylcarbonyle ;
un groupe alcoxy en C₁ à C₁₀ linéaire ou ramifié ou en C₃ à C₇ cyclique contenant éventuellement une ou plusieurs doubles liaisons ;
un groupe arylalcoxycarbonyle ;
un groupe amino ;
R₄ est :
un groupe hydroxyle, excepté lorsque R₂ ou R₃ représente un atome d'hydrogène ou un groupe acétyle ;
un groupe alcoxy en C₁ à C₂₀ linéaire ou ramifié ou en C₃ à C₇ cyclique contenant éventuellement une ou plusieurs doubles liaisons ;
un groupe alcoxycarbonyloxyalcoxy ou alkylcarbonyloxyalcoxy portant un groupe alkyle linéaire, ramifié ou cyclique ;
un groupe arylalcoxy.

3. Composés tels que revendiqués selon la revendication 2 dans lesquels R₄ est :
un groupe hydroxyle, excepté lorsque R₂ ou R₃ représenté un atome d'hydrogène ou un groupe acétyle,
un groupe alcoxy en C₄ à C₁₆ linéaire ou ramifié ou en C₃ à C₇ cyclique, contenant éventuellement une ou plusieurs doubles liaisons.

4. Composés tels que revendiqués selon la revendication 3 dans lesquels R₄ est un groupe alcoxy en C₄ à C₁₀ linéaire ou ramifié.

5. Composé tel que revendiqué selon les revendications 1 à 3 sélectionné parmi :
l'ester éthylique de la D-carnosine et ses sels
l'ester octylique de la D-carnosine et ses sels
l'ester méthylique de la D-carnosine et ses sels
l'ester n-butylique de la D-carnosine et ses sels
l'ester éthylique de la N-acétyl-D-carnosine
l'ester méthylique de la N-acétyl-D-carnosine,
l'ester octylique de la N-acétyl-D-carnosine
la N-Eoc-D-carnosine et ses sels
l'ester méthylique de la N-Eoc-D-carnosine
l'ester benzylique de la N-Eoc-D-carnosine
l'ester de D-carnosine-1-(éthoxycarbonytoxy)éthyte
l'ester de Eoc-D-carnosine-1-(éthoxycarbonyloxy)éthyle la Z-D carnosine et ses sels
l'ester méthylique de la Z-D carnosine
l'ester octylique de la Z-D carnosine.

6. Composition contenant une quantité efficace d'un ou plusieurs composés tels que revendiqués selon les revendications 1 à 5 en combinaison avec des excipients appropriés.
